(19) Europäisches Patentamt
European Patent Office
Office européen des brevets

(11) Publication number: 0 268 424 B1

# (12) EUROPEAN PATENT SPECIFICATION

(45) Date of publication of patent specification: 10.07.91

(51) Int. Cl.5: C07C 233/49, C07C 231/00

(21) Application number: 87309990.7

(22) Date of filing: 12.11.87

## (54) A process for preparing a polymerisable latex-forming solution incorporating unsaturated nitrogen-containing acid.

(30) Priority: 14.11.86 US 930485

(43) Date of publication of application:
25.05.88 Bulletin 88/21

(45) Publication of the grant of the patent:
10.07.91 Bulletin 91/28

(84) Designated Contracting States:
BE DE FR GB IT LU NL

(56) References cited:
EP-A- 0 002 254
EP-A- 0 020 000
DE-A- 2 640 615
DE-B- 1 297 598
US-A- 4 443 623

CHEMICAL ABSTRACTS, vol. 100, no. 23, 4 June 1984, Columbus, Ohio, US; AMERICAN CYANAMID CO.: "Alkyl acrylamidoglycolates and their alkyl esters", p. 539, no. 191398b, right column; & JP-A-58 206546 & CA-A-1 209 583

(73) Proprietor: GENCORP INC.
175 Ghent Road
Akron Ohio 44313(US)

(72) Inventor: White, Woodrow W.
672 Mull Avenue, Apt. 2B
Akron, OH 44313(US)

(74) Representative: Armitage, Ian Michael et al
MEWBURN ELLIS & CO. 2/3 Cursitor Street
London EC4A 1BQ(GB)

EP 0 268 424 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to the preparation of polymerisable solutions incorporating unsaturated nitrogen-containing acid.

Heretofore, unsaturated nitrogen-containing acids have been prepared using alkali compounds as catalyst. The end product was generally therefore a basic salt which required purification e.g. using an acid such as hydrochloric, to neutralize the solution containing the end product which was thus precipitated.

In the prior art, US-A-3,422,139 relates to the preparation of acrylamidoglycolic acid using an alkali carbonate catalyst, and describes the need to crystallize the product before use.

The problem addressed herein is to provide a new preparation of a polymerisable latex-forming solution, which may provide improved latex properties.

The invention provides a process for preparing a polymerisable latex-forming solution, characterised in that an unsaturated nitrogen-containing acid of the formula

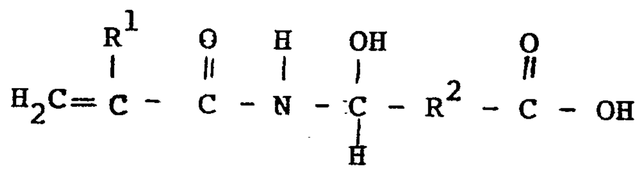

in which $R^1$ is H or an alkyl having from 1 to 3 carbon atoms, and $R^2$ is $CH_2$, $C_2H_4$ or absent,

is prepared by reacting an unsaturated amide of the formula

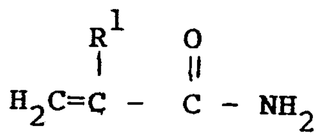

with an aldehyde acid of the formula

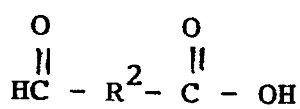

in the absence of basic catalyst in an aqueous medium having a pH of from about 1.2 to about 7.0,

in the proportion of 1.2-2.4 equivalents of the unsaturated amide to one equivalent of the aldehyde acid, to produce the nitrogen-containing acid in a salt-free form which is incorporated in the polymerisable latex-forming solution.

In another aspect the invention provides polymerisable latex-forming solutions obtainable by the above process.

The excess of amide over aldehyde acid in the reaction is desirable inasmuch as it speeds up the reaction rate. As stated, from about 1.2 to about 2.4, preferably from about 1.7 to about 2.0, equivalents of amide are used for each equivalent of aldehyde acid.

Also, improved latex properties can result from the presence of an unsaturated amide such as acrylamide. Such amide can conveniently be introduced as excess in the preparation of the unsaturated nitrogen-containing acid.

The reaction of the reactants is generally carried out in the aqueous reaction medium so that a solution is produced. Although the solution can contain generally any amount of solid, unsaturated nitrogen-containing acid therein, it may be from about 25% to about 70% by weight of solids, desirably from about 30% to about 60% by weight, and most preferably from about 40% to about 55% by weight based upon the total weight of the solution, that is the weight of the solid acid product and the liquid. The reaction is generally carried out at elevated temperatures with higher temperatures yielding faster reaction rates of the

reactants. Generally, the reaction temperature is at least 40° C. Since water is generally used, as the reaction medium, the maximum temperature will be 100° C unless the reaction is carried out under pressure. A desirable reaction temperature range is from about 60° C to about 100° C, with from about 70° C to about 100° C being preferred. The reaction is generally carried out at atmospheric pressure because of convenience although either subatmospheric or superatmospheric pressures can also be utilized.

As noted above, the reaction is conducted without the use of any salt-forming basic catalyst, and preferably without buffer systems and the like, such that a salt-free unsaturated nitrogen containing acid is produced. This method not only produces a product having low impurities, but is also relatively inexpensive since purification is not required. Inasmuch as a basic catalyst and the like are not utilized the pH of the reaction as well as of the slurry is relatively low or acidic and is from about 1.2 to about 7.0, and preferably from about 1.4 to about 4.0.

An advantage of the present invention is that since the unsaturated nitrogen-containing acid is produced in a relatively pure solution, it can be utilized as it is, without any purification of the solution, in various latex polymerization reactions well known to the art and to the literature.

The reaction between the unsaturated amide and the aldehyde acid produces an unsaturated nitrogen-containing acid of the following formulation;

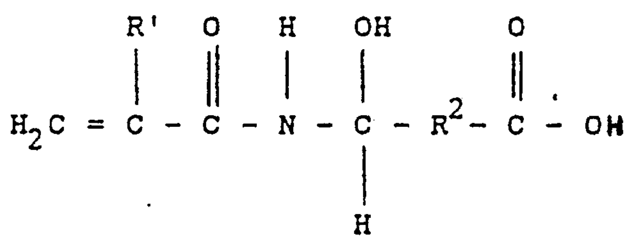

wherein $R^1$ and $R^2$ are as set forth hereinabove. Examples of suitable products include methacrylamidoglycolic acid, acrylamido lactic acid, with acrylamidoglycolic acid being preferred.

Various conventional additives e.g. inhibitors, can be utilized during the reaction as known to the art and to the literature. Inhibitors are utilized to prevent polymerization of the vinyl group in the unsaturated amide. Generally, free radical inhibitors are utilized such as hydroquinone, para-methoxy phenol, and t-butyl catechol. When utilized, the amount of the inhibitors is generally from about 10 to about 1,000 parts by weight, desirably from about 20 to about 500 parts by weight and preferably from about 5 to about 150 parts by weight for each 1 million parts by weight of the reactants.

The unsaturated nitrogen-containing acids are generally prepared in a suitable vessel, such as a reactor, etc., and subsequently utilized in solution form and added to a solution containing latex forming monomers. Alternatively, the reactants can be reacted in-situ in a latex forming vessel. That is, the unsaturated amide and the aldehyde acid can be added to a vessel containing a latex forming monomer solution as the reaction medium and reacted therein while the latex monomers are being polymerized. Upon formation of the unsaturated nitrogen-containing acid it will generally react with the latex forming monomers. It is thus to be understood that such in situ preparation is within the scope of the present invention. The latex-forming monomers can be any conventional monomers known to the art as well as to the literature and include monomers such as the various conjugated dienes containing from 4 to 10 carbon atoms, the various vinyl substituted aromatics containing from 8 to 12 carbon atoms, such as styrene, and the like.

The invention will be better understood by reference to the following Examples:

EXAMPLE I

This example illustrates the effect of pH of the reaction media on rate of disappearance of glyoxylic acid and the subsequent effect on cure rate of the saturated Whatman paper.

Three reactions were carried out at 50° C using the following formulations:

| | A | B | C |
|---|---|---|---|
| 53.7% glyoxylic acid, gms | 94.6 | 94.6 | 94.6 |
| 50% acrylamide, gms | 178 | 178 | 178 |
| 50% sodium hydroxide, gms | 0 | 38.5 | 62.5 |
| pH | 1.6 | 3.5 | 7.0 |

The samples were tested for residual glyoxylic acid each hour with results as follows:

| REACTION TIME - HOURS | RESIDUAL GLYOXYLIC ACID - % | | |
|---|---|---|---|
| 1 | (117.6) | 62.5 | 18.2 |
| 2 | 94.9 | 45.5 | 14.0 |
| 3 | 85.2 | 44.2 | 14.5 |
| 4 | 66.4 | 32.2 | 13.1 |
| 5 | 48.2 | 37.5 | 11.6 |
| 6 | 40.5 | 24.3 | |
| Overnight | 27.3 | 23.4 | |

It is apparent that the test method should have been recalibrated since A after 1 hour is still greater than 100%. However, it is clear that the reaction is accelerated at higher pH's.

CHEMICAL DEFINITIONS:

| | |
|---|---|
| Hampene Na-3 | A 38% aqueous solution of Trisodium ethylene diamine tetra acetate<br>W. R. Grace & Co. |
| Dowfax 2A-1 | Light colored liquid containing 45% minimum active ingredients in water of sodium dodecyl diphenyl oxide disulfonate<br>$C_{12}H_{25}C_{12}H_7O\ (SO_3Na)_2$<br>The Dow Chemical Co., an anionic surfactant |

| | |
|---|---|
| Aerosol MA | Bis(1-methylamyl) sodium sulfosuccinate<br>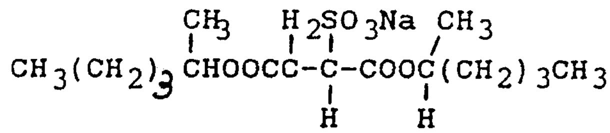<br>American Cyanamid Co., an anionic surfactant. 80% in water. |
| Sipex BOS | Sodium 2-ethyl hexyl sulfate<br>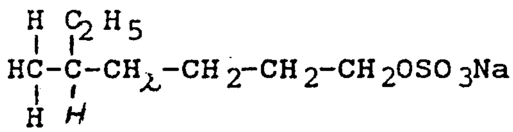<br>Alcolac Inc., 40% aqueous solution. |
| Sulfole 120 | t-dodecyl mercaptan, avg. mol. wt. 198<br>Calc. purity wt. 96.8% and<br>mercaptan sulfur wt. 15.4%<br>Phillips Petroleum, Co., Rubber Div. |

The test method utilized in the examples for measuring wet tensile strength is as follows:

Whatman #4 chromatographic paper die-cut to 10 x 25cm (8" x 10") is used with a tolerance of ±5.0% in an untreated weight basis. The sheets are saturated with the latex using a laboratory padder at a binder add-on target of 20 ± 1.5% based on the finished sheet weight. The saturated sheets are air dried at n room temperature and then duplicate sheets are cured on the steam heated drier can at 157°C (315°F) for 15, 30, 60, and 300 seconds. All sheets are conditioned in accordance with TAPPI test method T402 before testing. Then they are immersed in distilled water until they are completely wetted. The tensile is then determined on four test strips in both the machine direction (MD) and in the cross direction (CD) for each cured sheet. The average of the eight results in each direction and the total tensile is reported as the sum of the CD and MD averages. This result is normalized to a basis weight of 33kg/91m (73.0 lbs./300 ft.) and a binder add on of 20%.

EXAMPLE II

Each of the products in Example 1 was used at 7 PHM in polymerization of latexes in bottles as detailed below. In each case an initial charge was made to the bottles using the ingredients in the order listed in the table. The bottles were flushed with nitrogen, capped, and allowed to react 45 minutes at 65°C.

The bottles were opened, recharged with the appropriate amount of styrene, Sulfole 120, and butadiene as listed in the table. This charge was reacted until the bottles were under vacuum as a result of most of the monomers having reacted.

The bottles were opened a second time and further monomers, Sulfole 120, and the experimental activated acids added as listed. This charge was also reacted to vacuum. The latexes obtained were tested without stripping.

| | LATEX A | B | C |
|---|---|---|---|
| INITIAL CHARGE | | | |
| Water, ml | 68 | 68 | 68 |
| Solution A, ml | 20 | 20 | 20 |
| Solution B, ml | 60 | 60 | 60 |
| Solution C, ml | 18 | 18 | 18 |
| Styrene, gms | 15 | 15 | 15 |
| Reacted 45 minutes at 65 °C | | | |
| FIRST CHARGE BACK | | | |
| Styrene, gms | 34 | 34 | 34 |
| Solution D, gms | 10 | 10 | 10 |
| Butadiene, gms | 40 | 40 | 40 |
| Reacted to vacuum | | | |
| SECOND CHARGE BACK | | | |
| Water, ml | 0 | 57 | 57 |
| Solution E, gms | 93 | 0 | 0 |
| Solution F, gms | 0 | 27, | 0 |
| Solution G, gms | 0 | 0 | 27 |
| Solution C, ml | 10 | 10 | 10 |
| Styrene, gms | 34 | 34 | 34 |
| Solution D, gms | 10 | 10 | 10 |
| Butadiene, gms | 40 | 40 | 40 |
| Reacted to vacuum | | | |

Solution A - 0.5% (W/V) Hampene Na-3, 0.9% (W/V) Dowfax 2A-1, 5.0% (W/V) Sipex BOS, and 3.0% (W/V) Aerosol MA in deionized water
Solution B - 5.0% (W/V) Itaconic acid in deionized water
Solution C - 5.0% (W/V) Sodium persulfate in deionized water
Solution D - 3.8% (W/W) Sulfole 120 in styrene
Solution E - 15.0% (W/W) A from Example 1 in deionized water
Solution F - Product B from Example 1 in deionized water
Solution G - Product C from Example 1 in deionized water

LATEX PROPERTIES

| | A | B | C |
|---|---|---|---|
| Total Solids, % | 43.8 | 44.6 | 44.7 |
| pH | 2.4 | 3.7 | 4.4 |
| Brookfield Viscosity, cps | 43 | 48 | 45 |
| Surface Tension, dynes, cm | 45.7 | 45.7 | 44.7 |

It is evident that the three latexes have different pH's as a result of the differences in pH of the r eaction to form the products as shown in Example 1.

Each of the latexes were tested in Whatman #4 paper with the following results:

| CURE TIME - SECONDS | | NWTT | |
|---|---|---|---|
| 15 | 24.6 | 18.3 | 15.0 |
| 30 | 27.1 | 23.2 | 18.8 |
| 60 | 28.7 | 26.6 | 22.4 |

Example A relates to the present invention in that no basic catalyst such as sodium hydroxide was utilized. In contrast, Examples B and C relate to the prior art method of utilizing a basic catalyst. Even though the reaction between glyoxylic acid and acrylamide is promoted by a high pH, the above data indicates that such increased pH results in poor latex performance. Thus, the product of the present invention produces better properties on the Whatman #4 paper test than the same prior art solution made with sodium hydroxide.

EXAMPLE III

This example shows the preparation of a product at 49.5 percent total solids and having a mole ratio of 1:1.8 of glyoxylic acid to acrylamide. This product when used in an emulsion polymerization recipe at 7 PHM would be theoretically equivalent to 5 PHM of acrylamidoglycolic acid and 2 PHM of acrylamide.

Three (3) polymerization bottles were each charged with 143.5 grams (1.03 moles) of 53.3% (W/W) of glyoxylic acid, 267 grams (1.88 moles) of 50.0% (W/W) of acrylamide, and 17.5 grams (25 PPM on AGA) of a 0.03% (W/V) solution of paramethoxy phenol in deionized water. The bottles were rotated in a polymerization bath at 77° C (170° F) for five (5) hours. Alternate bottles were tested for residual glyoxylic acid at 1 1/2 hour intervals with the results of 34.9, 28.8, and 18.4 percent respectively. The contents of the bottles were blended and stored in a refrigerator.

It is noted that the theoretical total solids content of this product is 49.5 percent. The commercial product acrylamidoglycolic acid monohydrate is only soluble at about 13 percent. The reason for this wide discrepancy in solubility is unknown but the greater solubility of the product of the present invention is a significant commercial advantage since it enables preparation of latexes having a high solids content.

EXAMPLE IV

The product prepared in Example III was used at 3, 5, and 7 PHM, in polymerization of latexes in one gallon reactors equipped with stirrer, heating and cooling means, charging and discharging means, and temperature controller. The reactor was purged with nitrogen before use and the polymerization was conducted under nitrogen.

## PROCESS

A. INITIAL CHARGE TO REACTOR (Same for Latexes A, B, and C)

| | AS IS PARTS | PURE PARTS |
|---|---|---|
| Water | 88.7 | 90.0 |
| Hampene Na-3 | 0.132 | 0.05 |
| Dowfax 2A-1 | 0.567 | 0.085 |
| Sipex BOS | 1.18 | 0.5 |
| Aerosol MA | 0.375 | 0.3 |
| Itaconic Acid | 1.5 | 1.5 |
| Sodium Persulfate | 0.45 | 0.45 |
| Styrene | 7.5 | 7.48 |

Line temperature out at 65°C and pull vacuum

React 45 minutes

B. FIRST MONOMER CHARGE

| | A | | B | | C | |
|---|---|---|---|---|---|---|
| | AS IS | PURE | AS IS | PURE | AS IS | PURE |
| Styrene | 7.333 | 7.311 | 7.666 | 7.643 | 8.000 | 7.976 |
| Sulfole 120 | 0.063 | 0.063 | 0.063 | 0.063 | 0.063 | 0.063 |
| Butadiene | 6.667 | 6.647 | 6.667 | 6.647 | 6.667 | 6.647 |

React to about 75% conversion of monomers

C. Second Monomer Charge (Same as First)

D. Third Monomer Charge (Same as First and Second)

E. Fourth Monomer Charge

| | A | | B | | C | |
|---|---|---|---|---|---|---|
| | AS IS | PURE | AS IS | PURE | AS IS | PURE |
| Water | 9.64 | 12 | 10.32 | 12 | 10.99 | 12 |
| Example 3 | 4.67 | 2.31 | 3.33 | 1.65 | 2.00 | 0.99 |
| Styrene | 7.333 | 7.311 | 7.666 | 7.643 | 8.000 | 7.976 |
| Sulfole 120 | 0.063 | 0.063 | 0.063 | 0.063 | 0.063 | 0.063 |
| Butadiene | 6.667 | 6.647 | 6.667 | 6.647 | 6.667 | 6.647 |

React to 75% conversion of monomers

F. Fifth Monomer Charge

| | A | | B | | C | |
|---|---|---|---|---|---|---|
| | AS IS | PURE | AS IS | PURE | AS IS | PURE |
| Water | 9.64 | 12 | 10.32 | 12 | 10.99 | 12 |
| Example 3 | 4.67 | 2.31 | 3.33 | 1.65 | 2.00 | 0.99 |
| Water | 2.75 | 3 | 2.75 | 3 | 2.75 | 3 |
| Sodium Persulfate | 0.25 | 0.25 | 0.25 | 0.25 | 0.25 | 0.25 |
| Styrene | 7.333 | 7.311 | 7.666 | 7.643 | 8.000 | 7.976 |
| Sulfole 120 | 0.063 | 0.063 | 0.063 | 0.063 | 0.063 | 0.063 |
| Butadiene | 6.667 | 6.647 | 6.667 | 6.647 | 6.667 | 6.647 |

React to 75% conversion of monomer

G. Sixth Monomer Charge (Same as fifth)

React to constant solids plus one hour

H. Post Polymerization - Short Stop for Each Batch

| | AS IS | PURE |
|---|---|---|
| Water | 2,974 | 3 |
| Diethylhydroxyl Amine | 0.176 | 0.15 |

All latexes were stable during polymerization and had the following properties:

| | A | B | C |
|---|---|---|---|
| Total Solids,% | 41.9 | 41.9 | 43.1 |
| pH | 2.7 | 2.5 | 2.7 |
| Viscosity Brookfield, cps | 173 | 39 | 20 |
| Surface Tension, dynes, cm | 48.6 | 49.6 | 47.6 |

The latexes were used to saturate Whatman #4 chromatographic paper as described in the Test Method for measuring wet tensile development and the results are set forth in the following table. Results obtained with a conventional commercial latex (D).

| | NWTT 2 73# & 20.0% | | | |
|---|---|---|---|---|
| Cure Time, Seconds | A | B | C | D |
| 15 | 28.0 | 26.4 | 22.7 | 12.0 |
| 30 | 29.6 | 26.1 | 24.9 | 15.1 |
| 60 | 31.9 | 30.8 | 26.3 | 17.4 |
| 300 | 36.1 | 32.2 | 28.0 | 20.9 |

GENERAL FUNCTIONAL COMPOSITION, PHM

| | A | B | C | D |
|---|---|---|---|---|
| Example 3 | 7 | 5 | 3 | 0 |
| Acrylamidoglycolic Acid | 0 | 0 | 0 | 0 |
| Acrylamide | 0 | 0 | 0 | 0 |

THEORETICAL EQUIVALENTS

| | A | B | C | D |
|---|---|---|---|---|
| Acrylamidoglycolic Acid | 5 | 3.6 | 2.1 | 0 |
| Acrylamide | 2 | 1.4 | 0.9 | 0 |

The superior results for the latexes of this invention are clearly evident by comparing the results for A, B, and C with that of D. Latex C which contains only 3 PHM of the experimental reactive acid gave wet tensiles about 80 percent higher than those with the commercial latex D. Latex A which contains 7 PHM of the experimental reactive acid gave results about 126 percent higher than that of the commercial latex D.

**Claims**

1. A process for preparing a polymerisable latex-forming solution, characterised in that an unsaturated nitrogen-containing acid of the formula

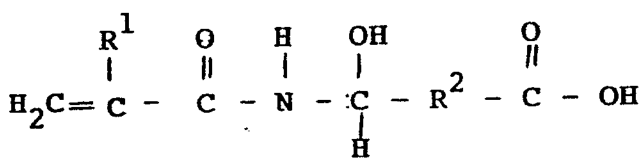

in which $R^1$ is H or an alkyl having from 1 to 3 carbon atoms, and $R^2$ is $CH_2$, $C_2H_4$ or absent, is prepared by reacting an unsaturated amide of the formula

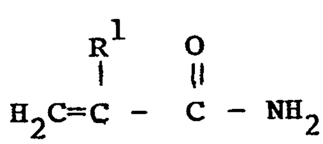

with an aldehyde acid of the formula

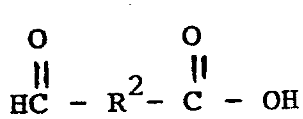

in the absence of basic catalyst, in an aqueous medium having a pH of from about 1.2 to about 7.0,

in the proportion of 1.2-2.4 equivalents of the unsaturated amide to one equivalent of the aldehyde acid, to produce the nitrogen-containing acid in a salt-free form which is incorporated in the polymerisable latex-forming solution.

**2.** A process according to claim 1 in which the prepared nitrogen-containing acid is added to a solution of latex-forming monomers.

**3.** A process according to claim 1 or claim 2 in which the proportion of the unsaturated amide is from about 1.4 to about 2 equivalents to each equivalent of aldehyde acid.

**4.** A process according to any one of the preceding claims in which the temperature of the reaction forming the nitrogen-containing acid is from about 60 °C to about 100 °C.

**5.** A process according to claim 4 in which said temperature is at least about 70 °C.

**6.** A process according to any one of the preceding claims in which $R^2$ is absent.

**7.** A process according to any one of the preceding claims in which the pH of said aqueous medium is from about 1.4 to about 4.0.

**8.** A process according to any one of the preceding claims in which $R^1$ is H.

**9.** A process according to any one of the preceding claims in which the nitrogen-containing acid forms a solution in the aqueous reaction medium containing from about 25 to about 70 percent by weight of dissolved nitrogen-containing acid based on the weight of said acid and said aqueous medium.

**10.** A process according to claim 9 in which the solution contains from about 30 to about 60 percent, more preferably from about 40 to about 55 percent, by weight of the nitrogen-containing acid.

**11.** A process according to any one of the preceding claims in which the polymerisable latex includes conjugated diene monomers of from 4 to 10 carbon atoms, and/or vinyl aromatic monomers of from 8 to 12 carbon atoms.

**12.** A polymerisable latex-forming solution containing an unsaturated nitrogen-containing acid, obtainable by a process according to any one of claims 1 to 11.

**Revendications**

**1.** Procédé pour préparer une solution polymérisable formant un latex, caractérisé en ce qu'on prépare un acide insaturé contenant de l'azote de la formule

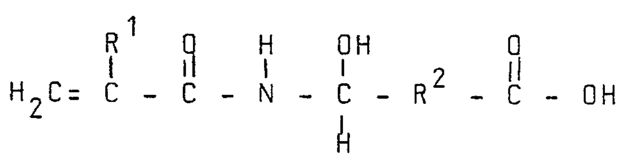

où $R^1$ est H ou un alkyle ayant de 1 à 3 atomes de carbone et $R^2$ est $CH_2$, $C_2H_4$ ou est absent,

par réaction d'un amide insaturé de la formule

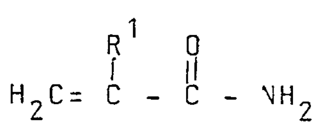

avec un aldéhyde acide de la formule

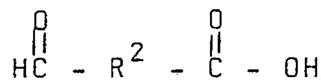

en l'absence d'un catalyseur basique dans un milieu aqueux ayant un pH d'environ 1 ,2 à environ 7,0,

à la proportion de 1,2-2,4 équivalents de l'amide insaturé pour un équivalent de l'aldéhyde acide, pour produire l'acide contenant de l'azote sous une forme sans sel qui est incorporé dans la solution polymérisable formant un latex.

2. Procédé selon la revendication 1 où l'acide préparé contenant de l'azote est ajouté à une solution de monomères formant un latex.

3. Procédé selon la revendication 1 ou la revendication 2 où la proportion de l'amide insaturé est comprise entre environ 1,4 et environ 2 équivalents pour chaque équivalent de l'aldehyde acide.

4. Procédé selon l'une quelconque des revendications précédentes où la température de la réaction formant l'acide contenant de l'azote est comprise entre environ 60 ° C et environ 100 ° C.

5. Procédé selon la revendication 4 où ladite température est d'au moins environ 70 ° C.

6. Procédé selon l'une quelconque des revendications précédentes où $R^2$ est absent.

7. Procédé selon l'une quelconque des revendications précédentes où le pH dudit milieu aqueux est compris entre environ 1,4 et environ 4,0.

8. Procédé selon l'une quelconque des revendications précédentes où $R^1$ est H.

9. Procédé selon l'une quelconque des revendications précédentes où l'acide contenant de l'azote forme une solution dans le milieu réactionnel aqueux contenant environ 25 à environ 70 pour cent en poids d'acide contenant de l'azote dissous en se basant sur le poids dudit acide et dudit milieu aqueux.

10. Procédé selon la revendication 9 où la solution contient environ 30 à environ 60 pour cent, mieux environ 40 à environ 55 pour cent en poids de l'acide contenant de l'azote.

11. Procédé selon l'une quelconque des revendications précédentes où le latex polymérisable contient des monomères diènes conjugués de 4 à 10 atomes de carbone et/ou des monomères vinyliques aromatiques de 8 à 12 atomes de carbone.

12. Solution formant un latex polymérisable contenant un acide insaturé contenant de l'azote que l'on peut obtenir par le procédé selon l'une quelconque des revendications 1 à 11.

**Ansprüche**

1. Verfahren zur Herstellung einer polymerisierbaren Latexbildenden Lösung, dadurch gekennzeichnet, daß eine ungesättigte, Stickstoff enthaltende Säure der Formel

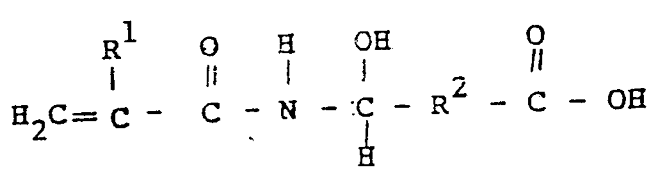

worin $R^1$ für H oder eine Alkylgruppe mit 1 bis 3 Kohlenstoffatomen steht und $R^2$ $CH_2$, $C_2H_4$ bedeutet oder fehlt,

hergestellt wird durch Umsetzung eines ungesättigten Amids der Formel

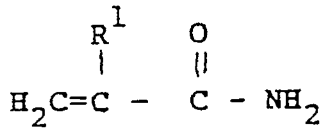

mit einer Aldehydsäure der Formel

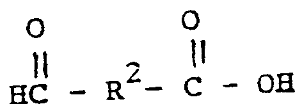

in Abwesenheit eines basischen Katalysators in einem wäßrigen Medium mit einem pH-Wert von etwa 1,2 bis etwa 7,0 in einem Mengenverhältnis von 1,2 bis 2,4 Äquivalenten des ungesättigten Amids zu einem Äquivalent der Aldehydsäure unter Bildung der Stickstoff enthaltenden Säure in einer salzfreien Form, die der polymerisierbaren Latex-bildenden Lösung einverleibt wird.

**2.** Verfahren nach Anspruch 1, worin die hergestellte Stickstoff enthaltende Säure einer Lösung von Latex-bildenden Monomeren zugesetzt wird.

**3.** Verfahren nach Anspruch 1 oder 2, worin der Mengenanteil des ungesättigten Amids etwa 1,4 bis etwa 2 Äquivalente pro Äquivalent der Aldehydsäure beträgt.

**4.** Verfahren nach einem der vorhergehenden Ansprüche, worin die Reaktionstemperatur zur Bildung der Stickstoff enthaltenden Säure etwa 60 bis etwa 100 °C beträgt.

**5.** Verfahren nach Anspruch 4, worin die Temperatur mindestens etwa 70 °C beträgt.

**6.** Verfahren nach einem der vorhergehenden Ansprüche, worin $R^2$ fehlt.

**7.** Verfahren nach einem der vorhergehenden Ansprüche, worin der pH-Wert des wäßrigen Mediums etwa 1,4 bis etwa 4,0 beträgt.

**8.** Verfahren nach einem der vorhergehenden Ansprüche, worin $R^1$ für H steht.

**9.** Verfahren nach einem der vorhergehenden Ansprüche, worin die Stickstoff enthaltende Säure eine Lösung bildet in dem wäßrigen Reaktionsmedium, das etwa 25 bis etwa 70 Gew.-% der gelösten, Stickstoff enthaltenden Säure enthält, bezogen auf das Gewicht von Säure und wäßrigem Medium.

**10.** Verfahren nach Anspruch 9, worin die Lösung etwa 30 bis etwa 60 Gew.-%, vorzugsweise etwa 40 bis etwa 55 Gew.-%, der Stickstoff enthaltenden Säure enthält.

**11.** Verfahren nach einem der vorhergehenden Ansprüche, worin der polymerisierbare Latex umfaßt konjugierte Dien-Monomere mit 4 bis 10 Kohlenstoffatomen und/oder vinylaromatische Monomere mit 8 bis 12 Kohlenstoffatomen.

**12.** Polymerisierbare Latex-bildende Lösung, die eine ungesättigte, Stickstoff enthaltende Säure enthält, wie sie nach dem Verfahren nach einem der Ansprüche 1 bis 11 erhältlich ist.